# EUROPEAN PATENT APPLICATION

(11) **EP 3 651 558 A1**
(43) Date of publication of application: **13.05.2020**
(21) Application number: 19207384.9
(22) Date of filing: 06.11.2019
(51) Int. Cl.: H05K 3/28, H05K 1/03

(54) **HERMETICALLY SEALED PRINTED CIRCUIT BOARDS**

(30) Priority: 07.11.2018 US 201862756776 P; 10.10.2019 US 201916598078
(71) Applicant: Covidien LP, Mansfield, MA 02048 (US)
(72) Inventor: SGROI, Anthony, Wallingford, CT Connecticut 06492 (US)
(74) Representative: Maschio, Antonio

(57) **Abstract**

A method of hermetically sealing electronic components (134) of a wiring harness (100) and integrating the wiring harness (100) into a surgical device (1) includes: positioning a tube (162) around a printed circuit board (130) and a first end section (110a) of a first flex cable (110) of a wiring harness (100) that is electrically coupled to a first end portion (130a) of the printed circuit board (130) at a first connection area such that a first end portion (162a) of the tube (162) extends axially beyond the first end section (110a) of the first flex cable (110) and a second end portion (162b) of the tube (162) extends axially beyond a second end portion (130b) of the printed circuit board (130); and filling at least a portion of the tube (162) with an encapsulate (164) to form a hermetic seal within the tube (162).

## Description

### TECHNICAL FIELD

This application claims the benefit of and priority to U.S. Provisional Patent Application Serial No. 62/756,776 filed November 7, 2018, the entire disclosure of which is incorporated by reference herein.

The present disclosure relates generally to reusable surgical devices. More particularly, the present disclosure relates to powered surgical devices with enhanced durability and increased moisture resistance.

### BACKGROUND

Powered surgical devices include electronic components, such as printed circuit boards, switches, sensors, etc., to enhance the control of functions of the surgical devices. The intelligence of such surgical devices result in a higher product cost compared to currently available disposable units. Accordingly, it would be beneficial if such intelligent devices are reusable.

Reusable surgical devices must be cleaned and sterilized prior to subsequent uses. Cleaning and sterilization procedures, however, are aggressive in nature. Cleaning (e.g., washing and/or disinfecting) utilizes alkaline solutions having high pH values (e.g., a pH of 11). Autoclaving (a common method of sterilization) utilizes high pressure superheated steam (e.g., 30 PSI @ 160°C for 20 minutes). Such environments are known to damage various electronic components. For example, surgical devices may suffer from moisture ingress during cleaning and/or sterilizing procedures which, in turn, may corrode and/or degrade the electronic components.

The electronic components of reusable surgical devices may be protected from high temperatures, steam, and/or moisture by utilizing, for example, conformal coatings, potting, sealed enclosures, and/or overmolding. The electronic components, however, may still suffer from moisture ingress during cleaning and/or sterilizing procedures (e.g., cracking or delamination of conformal coatings), and/or may be damaged during application of the protective materials (e.g., heat damage during sealing of enclosures).

Thus, it would be beneficial if the durability of the electronic components is enhanced to improve the reliability of the electronic components and/or extend the effective cycle life of the surgical devices.

### SUMMARY

The surgical devices of the present disclosure include a printed circuit board having electronic components and electrical connection areas housed within a hermetic sealing assembly. The electronic components and electrical connection areas are thus protected and configured to withstand environmental stresses associated with high pH cleaning and sterilization (e.g., autowashing and/or autoclaving), thereby rendering the printed circuit board more durable for re-use. Additionally, methods of the present disclosure include hermetically sealing and/or encapsulating the printed circuit board within the sealing assembly and integrating the sealed printed circuit board into a surgical device.

In an aspect of the present disclosure, a method of hermetically sealing electronic components of a wiring harness and integrating the wiring harness into a surgical device includes: positioning a tube around a printed circuit board and a first end section of a first flex cable of a wiring harness that is electrically coupled to a first end portion of the printed circuit board at a first connection area such that a first end portion of the tube extends axially beyond the first end section of the first flex cable and a second end portion of the tube extends axially beyond a second end portion of the printed circuit board; and filling at least a portion of the tube with an encapsulate to form a hermetic seal within the tube encasing the printed circuit board and the first end section of the first flex cable therein.

Positioning the tube may include covering a first end section of a second flex cable that is electrically coupled to the second end portion of the printed circuit board at a second connection area with the tube such that the second end portion of the tube extends axially beyond the first end section of the second flex cable. Filling the tube may include surrounding portions of the first and second flex cables extending through the first and second end portions of the tube with the encapsulate to seal the first and second end portions of the tube.

The method may further include applying a stimulus to cure the encapsulate within the tube. Applying the stimulus may include shining a light onto the tube, which is formed from a transparent polymer, to cure the encapsulate, which is a light cure resin.

The method may include placing the printed circuit board and the tube in an inert gas environment prior to positioning the tube around the printed circuit board.

The method may further include placing the wiring harness into an adapter assembly of a surgical device. The method may include electrically connecting a proximal electrical connector electrically coupled to a second end section of the first flex cable to a handle assembly of the surgical device and/or electrically connecting a distal electrical connector electrically coupled to a second end section of the second flex cable to an end effector of the surgical device.

In another aspect of the present disclosure, a wiring harness includes a printed circuit board, a first flex cable, a tube, and an encapsulate. The first flex cable includes a first end section electrically coupled to a first end portion of the printed circuit board at a first connection area. The tube is disposed around the printed circuit board and the first end section of the first flex cable. The tube has a first end portion extending laterally beyond the first connection area defined between the first flex cable and the printed circuit board, and a second end portion. The encapsulate is disposed within the first and second end portions of the tube and forms a hermetic seal within the tube encasing the printed circuit board and the first end section of the first flex cable therein.

The wiring harness may include a second flex cable including a first end section electrically coupled to a second end portion of the printed circuit board at a second connection area, the second end portion of the tube extending laterally beyond the second connection area.

A second end section of the first flex cable may be electrically coupled to a proximal electrical connector and/or a second end section of the second flex cable may be electrically coupled to a distal electrical connector.

The encapsulate may fill the entirety of the tube and surround the printed circuit board. The tube may be transparent and the encapsulate may be a light cure resin. The tube, filled with the encapsulate, may have a uniform thickness along the length thereof.

Other aspects, features, and advantages will be apparent from the description, drawings, and the claims.

### BRIEF DESCRIPTION OF THE DRAWINGS

Various aspects of the present disclosure are described herein below with reference to the drawings, which are incorporated in and constitute a part of this specification, wherein:
FIG. 1 is a perspective view of a surgical device in accordance with an embodiment of the present disclosure;
FIG. 2 is a perspective view of a wiring harness of an adapter assembly of the surgical device of FIG. 1;
FIG. 3 is a close-up view of a portion of the wiring harness of FIG. 2, illustrating a printed circuit board of the wiring harness disposed within a sealing assembly shown in phantom;
FIG. 4 is a top, perspective view of a portion of the wiring harness of FIG. 3, illustrating a hermetic tube of the sealing assembly positioned over the printed circuit board during assembly of the sealing assembly onto the wiring harness in accordance with an embodiment of the present disclosure; and
FIG. 5 is a top, perspective view of the portion of the wiring harness of FIG. 4, illustrating an encapsulate of the sealing assembly disposed within the hermetic tube.

### DETAILED DESCRIPTION

Surgical devices in accordance with embodiments of the present disclosure include a printed circuit board hermetically sealed within a sealing assembly to protect the printed circuit board from exposure to moisture during, for example, cleaning and/or sterilizing procedures where the surgical devices may be subjected to high temperatures, steam, chemicals, and/or moisture. The electronic components of the printed circuit board, as well as the electrical connections therebetween, are protected to prevent and/or resist breakdown over multiple/repeated cleaning and sterilizing cycles of the surgical device.

While the present disclosure is directed to rigid printed circuit boards (e.g., FR4 circuit boards), the principles of the present disclosure are equally applicable to a range of printed circuit boards (e.g., flexible printed circuit boards with or without a rigidizer), electronic components (e.g., sensors), and/or electronics assemblies housed within reusable surgical devices.

Embodiments of the present disclosure are now described in detail with reference to the drawings in which like reference numerals designate identical or corresponding elements in each of the several views. Throughout this description, the term "proximal" refers to a portion of a device, or component thereof, that is closer to a user, and the term "distal" refers to a portion of the device, or component thereof, that is farther from the user.

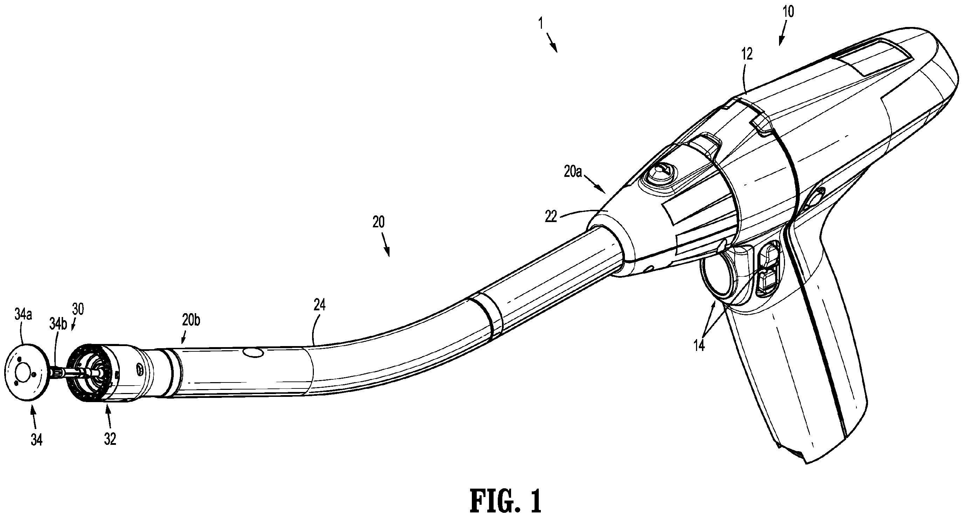

Turning now to FIG. 1, a surgical device 1 in accordance with an embodiment of the present disclosure is shown. The surgical device 1 is in the form of a powered handheld electromechanical surgical instrument, and includes a powered handle assembly 10, an adapter assembly 20, and a tool assembly or end effector 30. The powered handle assembly 10 is configured for selective connection with the adapter assembly 20 and, in turn, the adapter assembly 20 is configured for selective connection with the end effector 30.

The surgical device 1 will only further be described to the extent necessary to disclose aspects of the present disclosure. For a detailed description of the structure and function of exemplary surgical devices, reference may be made to commonly owned U.S. Patent Publication Nos. 2016/0296234 ("the '234 Publication") and 2016/0310134 ("the '134 Publication"), and U.S. Patent Application No. 15/972,606 ("the '606 Application"), the entire contents of each of which are incorporated herein by reference.

With continued reference to FIG. 1, the handle assembly 10 includes a handle housing 12 housing a power-pack (not shown) configured to power and control various operations of the surgical device 1, and a plurality of actuators 14 (e.g., finger-actuated control buttons, knobs, toggles, slides, interfaces, and the like) for activating various functions of the surgical device 1. The adapter assembly 20 has a proximal portion 20a including a knob housing 22 configured for operable connection to the handle assembly 10 and a distal portion 20b including an outer tube 24 configured for operable connection to the end effector 30. The end effector 30 including a loading unit 32 having a plurality of staples (not shown) disposed therein and an anvil assembly 34 including an anvil head 34a and an anvil rod 34b.

For a detailed description of exemplary handle assemblies, adapter assemblies, and end effectors which may be utilized in a surgical device of the present disclosure, reference may be made to the '234 and '134 Publications and the '606 Application, the entire contents of each of which were previously incorporated herein by reference.

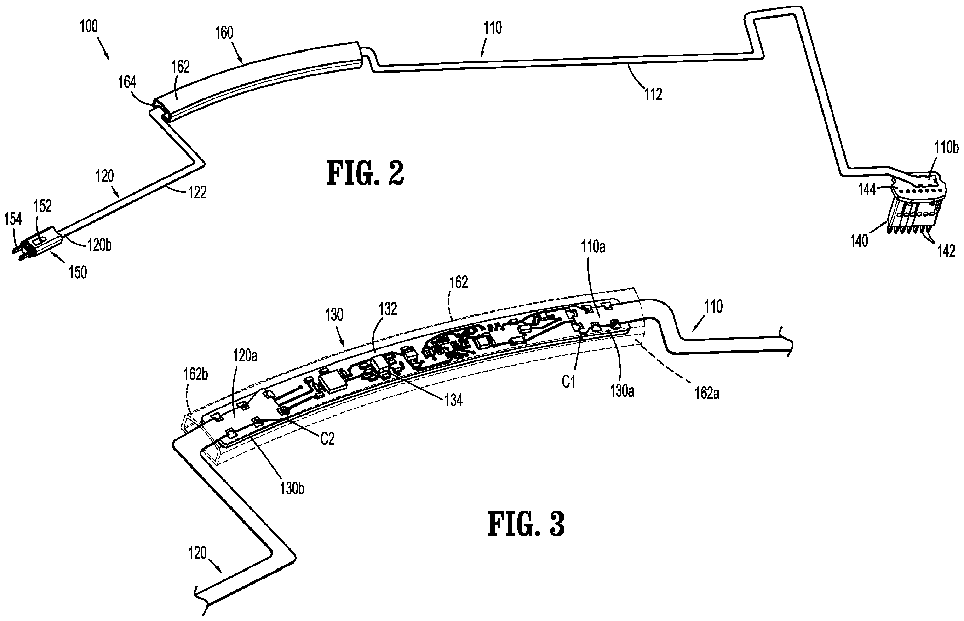

With reference now to FIG. 2, in conjunction with FIG. 1, the adapter assembly 20 includes a wiring harness 100 for electronically interconnecting the handle assembly 10 and the end effector 30 of the surgical device 1. The wiring harness 100 of the adapter assembly 20 is configured to enable communication between the handle assembly 10 and the end effector 30, and to relay power from the handle assembly 10 to the end effector 30. For example, this communication allows for calibration and communication of data and control signals between the end effector 30 and the adapter assembly 20, as well as between the adapter assembly 20 and the handle assembly 10, thereby transferring data pertaining to the end effector 30 to the handle assembly 10 and signals from the handle assembly 10 to the end effector 30.

As shown in FIGS. 2 and 3, the wiring harness 100 includes a first or proximal flex cable 110, a second or distal flex cable 120, and a printed circuit board 130 coupled to each of the first and second flex cables 110, 120. A proximal electrical connector 140 is coupled to the first flex cable 110 and a distal electrical connector 150 is coupled to the second flex cable 120 such that the first and second flex cables 110, 120 provide signaling paths to and from the printed circuit board 130 and electronic components (not shown) coupled to the proximal and distal electrical connectors 140, 150.

Each of the first and second flex cables 110, 120 includes a body or substrate 112, 122 fabricated from one or more layers or sheets of dielectric or insulative material, such as a polymer or a ceramic, and one or more layers of conductive material, such as a metal or a metal alloy, that form electrical traces (not explicitly shown) embedded within the insulative material. The electrical traces are exposed at first end sections 110a, 120a of the first and second flex cables 110, 120 for making an electrical connection with the printed circuit board 130, and at second end sections 110b, 120b of the first and second flex cables 110, 120 for making an electrical connection with the proximal and distal electrical connectors 140, 150.

The first and second flex cables 110, 120 are fabricated out of material(s) that are highly resistant to high pH environments (e.g., disinfecting chemicals such as KOH) and/or high temperatures and pressures (e.g., autoclave steam). The insulative material of the first and second flex cables 110, 120 may include, for example, liquid-crystal polymers (LCP), polyetherimide (PEI, such as those sold under the trademark ULTEM® of Sabic Global Technologies B.V.), polysulfone (PSU, such as those sold under the trademark UDEL® of Solvay Specialty Polymers USA, L.L.C.), polyphenylsulfone (PPSU, such as those sold under the trademark RADEL® of Solvay Specialty Polymers USA, L.L.C.), polyether ether ketone (PEEK), among other polymers (e.g., high temperature and/or high performance polymers) within the purview of those skilled in the art. The conductive material(s) of the first and second cables 110, 120 may be formed of, for example, copper, gold, silver, aluminum, platinum, and alloys thereof, among other electrically conductive materials within the purview of those skilled in the art.

The printed circuit board 130 includes a body or substrate 132 mechanically supporting and electrically connecting electronic components 134 thereon. The electronic components 134 may be, for example, surface mount technology and/or through-hole technology, including, for example, integrated circuits (e.g., microchips, microcontrollers, microprocessors), resistors, amplifiers, inductors, capacitors, sensing elements (e.g., optical sensors, pressure sensors, capacitive sensors), buttons, switches, circuit boards, electrical connectors, cables, and/or wires, among other elements or circuitry within the purview of those skilled in the art.

The substrate 132 is formed from at least one or more layers or sheets of dielectric of insulative material and one or more layers of conductive material that form conductive traces (not explicitly shown) in the substrate 132. Vias (not shown) may interconnect the conductive traces through different layers of the substrate 132. Electrical contact regions are disposed at terminal ends of the conductive traces of the substrate 132, and may include one or more pads (e.g., solder pads) to which the electronic components 134 are joined (e.g., soldered) or to which an electrical connection is made with the first and second flex cables 110, 120. While the printed circuit board 130 is shown including a rigid substrate 132 having an FR4 base and surface mount electronic components 134, other configurations are envisioned.

With continued reference to FIGS. 2 and 3, the first or distal end section 110a of the first flex cable 110 and the first or proximal end section 120a of the second flex cable 120 are electrically coupled (e.g., soldered) to opposed end portions 130a, 130b of the printed circuit board 130 at connection areas "C1," "C2" so that electrical connections are made to and from the printed circuit board 130. The second or proximal end section 110b of the first flex cable 110 is electrically coupled to the proximal electrical connector 140, and the second or distal end section 120b of the second flex cable 120 is electrically coupled to the distal electrical connector 150.

The proximal electrical connector 140 includes a plurality of electrical contact blades 142 supported on a printed circuit board 144 for mechanical and electrical connection to an electrical receptacle (not shown) of the handle assembly 10 (FIG. 1) which, in turn, is in electrical connection with a power-pack core assembly (not shown) that controls the various operations of the handle assembly 10 and thus, the surgical device 1. The distal electrical connector 150 includes a plug member 152 supporting a pair of arms 154 for mechanical and electrical connection with a chip assembly (not shown) of the end effector 30 (FIG. 1) that stores information about the end effector 30 and allows the handle assembly 10 to encode information thereto.

A sealing assembly 160 is disposed over the printed circuit board 130 as well as the connection areas "C1," "C2" between the first end sections 110a, 120a of the first and second flex cables 110, 120 and the end portions 130a, 130b of the printed circuit board 130 to hermetically seal the entire printed circuit board 130 as well as the first end sections 110a, 120a of the first and second flex cables 110, 120 therein. The sealing assembly 160 includes a sleeve or tube 162 positioned around the printed circuit board 130 and the first end sections 110a, 120a of the first and second flex cables 110, 120 such that ends 162a, 162b of the sleeve 162 extend axially beyond the connection areas "C1," "C2" between the first and second flex cables 110, 120 and the printed circuit board 130. The sealing assembly 160 also includes an encapsulating material or encapsulate 164 disposed within the tube 162 and sealing the ends 162a, 162b thereof to prevent moisture ingress into the tube 162.

The tube 162 is formed from material(s) that are highly resistant to high pH environments and/or high temperatures and pressures as discussed above with respect to the insulative material of the first and second flex cables 110, 120. The sleeve 160 may be formed from LCP, PEI, PPSU, PEEK, among other materials (e.g., high temperature and/or high performance polymers). The sleeve 162 may be formed from the same material or a different material as the first and/or second flex cables 110, 120.

The encapsulate 164 may be, for example, urethanes, acrylics, epoxies, among other materials that may form a solid, flexible bond so that the encapsulate 164 can move with the thermal and mechanical movement of the first and second flex cables 110, 120 and/or printed circuit board 130, and withstanding cleaning and sterilization cycles. The encapsulate 164 may be resins such as those sold under the trademark LOCTITE® of Henkel IP & Holding GMBH. The encapsulate 164 may be a sealant, such as a room-temperature vulcanization (RTV) silicone. The encapsulate 164 may be a conformal coating or potting material such as those sold under the trademarks HUMISEAL® of Columbia Chase Corporation, or DOLPHON® of John C. Dolph Company.

In embodiments, the encapsulate 164 cures upon application of a stimuli such as heat or moisture, or exposure to light (e.g., ultraviolet light). In such embodiments, the tube 162 is formed from a transparent material so that the encapsulate 164 can be cured within the tube 162. For example, a transparent polysulfone tube 162 may be used with an acrylic light cure resin encapsulate 164. In some embodiments, the encapsulate 164 is a multi-component system (e.g., a two-part system) in which the parts are kept isolated from one another and then combined to form the encapsulate 164.

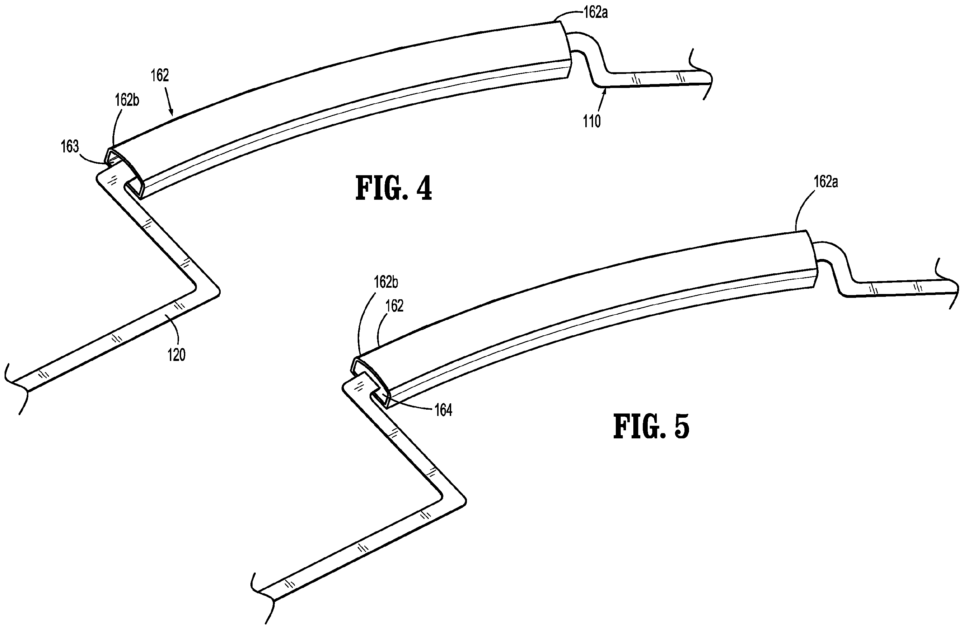

Referring now to FIGS. 4 and 5, a method of hermetically sealing the printed circuit board 130 of the wiring harness 100 in accordance with an embodiment of the present disclosure is shown. As seen initially in FIG. 4, in conjunction with FIG. 3, the tube 162 is positioned around the printed circuit board 130, as well as the first end sections 110a, 120a of each of the first and second flex cables 110, 120. The tube 162 defines an opening 163 longitudinally therethrough configured to receive the printed circuit board 130 therein. The tube 162 is axially longer than the printed circuit board 130 to also accommodate and cover the first end sections 110a, 120a of the first and second flex cables 110, 120 therein.

The tube 162 may be slid over one of the first or second flex cables 110, 120 towards the printed circuit board 130 or the tube 162 may be formed around the printed circuit board 130 by wrapping a sheet of material therearound and bonding the sheet of material to itself to form the tube 162. The tube 162 encircles the printed circuit board 130 as well as the first end sections 110a, 120a of the first and second flex cables 110, 120 such that the ends 162a, 162b of the tube 162 are offset an axial distance from the connection areas "C1," "C2" between the printed circuit board 130 and the first and second flex cables 110, 120.

The tube 162 is sized and shaped to have a complementary geometry with that of the printed circuit board 130 and the first end sections 110a, 120a of the first and second flex cables 110, 120. As shown, for example, in FIG. 4, the tube 162 has a curved configuration complementary to the curved configuration of the printed circuit board 130. It should be understood, however, that other configurations are envisioned that correspond with the size and shape of the printed circuit board 130.

As shown in FIG. 5, in conjunction with FIGS. 3 and 4, after the tube 162 is positioned over the printed circuit board 130, the opening 163 of the tube 162 is filled with the encapsulate 164 to seal the ends 162a, 162b of the tube 162 and form a moisture-proof barrier. The encapsulate 164 is rendered in a flowable (e.g., liquid) state for injecting or pouring the encapsulate 164 into the opening 163 of the tube 162 by any method suitable for the type of encapsulation material utilized, as is within the purview of those skilled in the art. For example, the encapsulate 164 may be mixed, blended, and/or heated to activate or make the encapsulate 164 flowable.

The encapsulate 164 may be passed through one of the ends 162a, 162b of the tube 162 to the other of the ends 162a, 162b such that the encapsulate 164 fills the entirety of the tube 162 (or at least a portion thereof) and covers or surrounds the printed circuit board 130 (e.g., extends completely around the printed circuit board 130). The size and shape of the opening 163 defined within the tube 162 may vary depending, for example, on the space needed to optimize protection of the printed circuit board 130 and the electronic components coupled thereto. Once filling is complete, the encapsulate 164 is allowed to solidify and/or cure, and the wiring harness 100 may be assembled into the adapter assembly 20. In embodiments utilizing an encapsulate that cures upon application of a stimuli, the appropriate stimuli may be applied to the sealing assembly 160. For example, in the case of light cure resins, light may be applied to the tube 162 to cure the encapsulate 164 therein.

In addition to filling the tube 162, the encapsulate 164 fills the ends 162a, 162b of the tube 162 (or at least portions thereof) and surrounds portions of the first and second flex cables 110, 120 exiting the tube 162 such that the sealing assembly 160 maintains a substantially uniform thickness along the length thereof (e.g., the ends 162a, 162b of the tube 162 and the encapsulate 164 terminate at the same thickness as the portion of tube 162 and encapsulate 164 covering the printed circuit board 130). This configuration removes the feathered edge associated with traditional coating processes thereby enhancing the bond between the encapsulate 164 and the first and second flex cables 110, 120, and improving resistance to peeling of the encapsulate 164 away from the first and second flex cables 110, 120 such as, for example, after several cleaning and autoclave cycles.

Assembly of the wiring harness 100, or portions thereof, may be performed using vacuum or in the presence of an inert gas (e.g., argon, nitrogen, etc.), as is within the purview of those skilled in the art. In embodiments, the electronic components 134 are assembled onto the printed circuit board 130 and/or the first and second flex cables 110, 120 are coupled to the printed circuit board 130 and sealed within the sealing assembly 160 in an inert gas environment, such as an inert glove box (e.g., a nitrogen-filled atmosphere), to ensure zero moisture content within the tube 162 of the sealing assembly 160.

In embodiments, a coating may be disposed over the electronic components 134 and/or the connection areas "C1," "C2" prior to sealing with sealing assembly 160. The coating may be a conformal coating that protects the electronic components 134 and/or connection areas "C1," "C2" against moisture and/or heat. Accordingly, the coating may act as an additional layer of protection in the event that the sealing assembly 160 should fail and moisture should permeate or ingress therethrough.

In embodiments, it is further contemplated that a moisture collection agent, e.g., a desiccant, may be provided within the tube 162, prior to filling the tube 162 with the encapsulate 164. For a detailed description of moisture collection agents suitable for use with the printed circuit boards of the present disclosure, reference may be made to U.S. Patent Application Serial No. 15/876,378, filed on January 22, 2018, the entire content of which is incorporated herein by reference.

It should be understood that the configuration of wiring harness (e.g., the printed circuit board and/or flex cables) may vary depending upon the desired functionality of the wiring harness, and one or more sealing assemblies may be utilized to protect electronic components and/or electrical connections of the wiring harness. It should also be understood that while the sealing assemblies are shown and described above as being part of a wiring harness disposed within an adapter assembly of the surgical device, the sealing assemblies may be utilized in other electronics assemblies and/or components of the surgical device, or other surgical devices.

Persons skilled in the art will understand that the structures specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. For example, the flexible cables of the present disclosure may be utilized in other surgical devices, such as robotic or powered surgical devices/instruments that are subject to sterilization procedures (e.g., autoclaving and/or autowashing). Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

The invention may be described by reference to the following numbered paragraphs:
1. A method of hermetically sealing electronic components of a wiring harness and integrating the wiring harness into a surgical device, the method comprising: positioning a tube around a printed circuit board and a first end section of a first flex cable of a wiring harness that is electrically coupled to a first end portion of the printed circuit board at a first connection area such that a first end portion of the tube extends axially beyond the first end section of the first flex cable and a second end portion of the tube extends axially beyond a second end portion of the printed circuit board; and filling at least a portion of the tube with an encapsulate to form a hermetic seal within the tube encasing the printed circuit board and the first end section of the first flex cable therein.
2. The method according to paragraph 1, wherein positioning the tube further includes covering a first end section of a second flex cable that is electrically coupled to the second end portion of the printed circuit board at a second connection area with the tube such that the second end portion of the tube extends axially beyond the first end section of the second flex cable.
3. The method according to paragraph 2, wherein filling the tube further includes surrounding portions of the first and second flex cables extending through the first and second end portions of the tube with the encapsulate to seal the first and second end portions of the tube.
4. The method according to paragraph 1, further comprising applying a stimulus to cure the encapsulate within the tube.
5. The method according to paragraph 4, wherein applying the stimulus includes shining a light onto the tube, which is formed from a transparent polymer, to cure the encapsulate, which is a light cure resin.
6. The method according to paragraph 1, further comprising placing the printed circuit board and the tube in an inert gas environment prior to positioning the tube around the printed circuit board.
7. The method according to paragraph 1, further comprising placing the wiring harness into an adapter assembly of a surgical device.
8. The method according to paragraph 1, further comprising electrically connecting a proximal electrical connector electrically coupled to a second end section of the first flex cable to a handle assembly of the surgical device.
9. The method according to paragraph 2, further comprising electrically connecting a distal electrical connector electrically coupled to a second end section of the second flex cable to an end effector of the surgical device.
10. A wiring harness comprising: a printed circuit board; a first flex cable including a first end section electrically coupled to a first end portion of the printed circuit board at a first connection area; a tube disposed around the printed circuit board and the first end section of the first flex cable, the tube having a first end portion extending laterally beyond the first connection area defined between the first flex cable and the printed circuit board, and a second end; and an encapsulate disposed within the first and second ends of the tube and forming a hermetic seal within the tube encasing the printed circuit board and the first end section of the first flex cable therein.
11. The wiring harness according to paragraph 10, further comprising a second flex cable including a first end section electrically coupled to a second end portion of the printed circuit board at a second connection area, the second end portion of the tube extending laterally beyond the second connection area.
12. The wiring harness according to paragraph 10, wherein a second end section of the first flex cable is electrically coupled to a proximal electrical connector.
13. The wiring harness according to paragraph 11, wherein a second end section of the second flex cable is electrically coupled to a distal electrical connector.
14. The wiring harness according to paragraph 10, wherein the encapsulate fills the entirety of the tube and surrounds the printed circuit board.
15. The wiring harness according to paragraph 10, wherein the tube is transparent and the encapsulate is a light cure resin.
16. The wiring harness according to paragraph 10, wherein the tube, filled with the encapsulate, has a uniform thickness along the length thereof.

## Claims

1. A method of hermetically sealing electronic components of a wiring harness and integrating the wiring harness into a surgical device, the method comprising:
positioning a tube around a printed circuit board and a first end section of a first flex cable of a wiring harness that is electrically coupled to a first end portion of the printed circuit board at a first connection area such that a first end portion of the tube extends axially beyond the first end section of the first flex cable and a second end portion of the tube extends axially beyond a second end portion of the printed circuit board; and
filling at least a portion of the tube with an encapsulate to form a hermetic seal within the tube encasing the printed circuit board and the first end section of the first flex cable therein.

2. The method according to claim 1, wherein positioning the tube further includes covering a first end section of a second flex cable that is electrically coupled to the second end portion of the printed circuit board at a second connection area with the tube such that the second end portion of the tube extends axially beyond the first end section of the second flex cable.

3. The method according to claim 2, wherein filling the tube further includes surrounding portions of the first and second flex cables extending through the first and second end portions of the tube with the encapsulate to seal the first and second end portions of the tube.

4. The method according to any preceding claim, further comprising applying a stimulus to cure the encapsulate within the tube; preferably wherein applying the stimulus includes shining a light onto the tube, which is formed from a transparent polymer, to cure the encapsulate, which is a light cure resin.

5. The method according to any preceding claim, further comprising placing the printed circuit board and the tube in an inert gas environment prior to positioning the tube around the printed circuit board.

6. The method according to any preceding claim, further comprising placing the wiring harness into an adapter assembly of a surgical device.

7. The method according to any preceding claim, further comprising electrically connecting a proximal electrical connector electrically coupled to a second end section of the first flex cable to a handle assembly of the surgical device.

8. The method according to claim 2, further comprising electrically connecting a distal electrical connector electrically coupled to a second end section of the second flex cable to an end effector of the surgical device.

9. A wiring harness comprising:
a printed circuit board;
a first flex cable including a first end section electrically coupled to a first end portion of the printed circuit board at a first connection area;
a tube disposed around the printed circuit board and the first end section of the first flex cable, the tube having a first end portion extending laterally beyond the first connection area defined between the first flex cable and the printed circuit board, and a second end; and
an encapsulate disposed within the first and second ends of the tube and forming a hermetic seal within the tube encasing the printed circuit board and the first end section of the first flex cable therein.

10. The wiring harness according to claim 9, further comprising a second flex cable including a first end section electrically coupled to a second end portion of the printed circuit board at a second connection area, the second end portion of the tube extending laterally beyond the second connection area.

11. The wiring harness according to claim 10, wherein a second end section of the first flex cable is electrically coupled to a proximal electrical connector.

12. The wiring harness according to claim 10, wherein a second end section of the second flex cable is electrically coupled to a distal electrical connector.

13. The wiring harness according to any of claims 9 to 12, wherein the encapsulate fills the entirety of the tube and surrounds the printed circuit board.

14. The wiring harness according to any of claims 9 to 13, wherein the tube is transparent and the encapsulate is a light cure resin.

15. The wiring harness according to any of claims 9 to 14, wherein the tube, filled with the encapsulate, has a uniform thickness along the length thereof.
